# EUROPEAN PATENT SPECIFICATION

(11) **EP 3 826 929 B1**
(45) Date of publication and mention of the grant of the patent: **06.09.2023**
(21) Application number: 19841965.7
(22) Date of filing: 23.07.2019
(51) Int. Cl.: B65D 5/04, B65D 75/52, B65D 81/02, B65D 85/20, B65D 85/30, B65D 85/60, A23L 21/25, B65D 77/00

(54) **SYSTEM AND METHOD FOR PACKAGING A SINGLE-SERVE PORTION OF HONEYCOMB**
SYSTEM UND VERFAHREN ZUM VERPACKEN EINER EINZELWABENPORTION
SYSTÈME ET PROCÉDÉ DE CONDITIONNEMENT D'UNE PORTION INDIVIDUELLE DE CONFISERIE HONEYCOMB

(30) Priority: 24.07.2018 US 201816044281
(43) Date of publication of application: 02.06.2021
(60) Divisional application: 23189039.3
(73) Proprietor: Honey, Feel Better LLC, Cardiff, California 92007 (US)
(72) Inventor: RAGGIO, Douglas Paul, Cardiff, California 92007 (US)
(74) Representative: Busana, Omar
(86) International application number: PCT/US2019/043011
(87) International publication number: WO 2020/023497

(56) References cited:
- EP-A2- 1 291 298
- DE-U1-202008 014 626
- GB-A- 2 418 655
- LU-A1- 81 493
- NL-C1- 1 030 706
- US-A- 5 897 011
- US-A1- 2011 127 319
- US-A1- 2014 272 005
- Mahako bees: "Comb honey = super food. How to cut and pack 100 gram samples", YouTube Daily Motion, 9 April 2014 (2014-04-09), page 1, XP054981407, Retrieved from the Internet: URL:https://www.dailymotion.com/video/x2oy qyj
- Anonymous: "Tennessee black truffle honeycomb", Regalis Foods , 29 December 2015 (2015-12-29), pages 1-3, XP055679513, Retrieved from the Internet: URL:www.regalisfoods.com/truffle-products- oils/tennessee-black-truffle-honeycomb [retrieved on 2019-09-09]
- Abdulwahid Ajibola: "Physio-chemical and physiological values of honey and its importance as a functional food", International Journal of Food and Nutrition Science, vol. 2, no. 6, 29 December 2015 (2015-12-29), pages 1-9, XP055679516, DOI: 10.15436/2377-0619.15.040

## Description

### FIELD OF THE DISCLOSURE

This disclosure relates to providing packaging for a single-serve portion of honeycomb. Honeycomb may interchangeably be referred to as "comb honey."

### BACKGROUND

Packaging food items in bulk is known. Packaging food items in single-serve portions is known. Individually wrapping smaller amounts of food is known: see LU81493A1 or GB2418655A or EP1291298A2.

### SUMMARY

Sales and consumption of honeycomb may increase if a conveniently-sized portion is individually packaged and/or wrapped. Honeycomb, potentially combined with one or more supplemental ingredients, may provide health-related and functional benefits to a consumer.

One aspect of the disclosure relates to a food package comprising a single-serve portion of honeycomb, a protective support structure, and/or other components. The phrase "single-serve" may be used interchangeably with the phrase "single serving". Honeycomb may be naturally produced by honey bees, and include a mass of hexagonal (prismatic) wax cells. In some implementations, a single-serve portion of honeycomb may have a shape of a cylinder, a rectangular prism, or a triangular prism. In some implementations, a single-serve-portion of honeycomb may be an amount that falls within at least one of the following three ranges: (i) a volume of the single-serve portion of honeycomb is between 0.5 and 6 cubic inches, (ii) a number of calories of the single-serve portion of honeycomb is between 50 and 500 calories, and (iii) a weight of the single-serve portion of honeycomb is between 20 and 200 grams. In some implementations, the protective support structure may be configured to protect the single-serve portion of honeycomb from impact. In some implementations, the protective support structure may be configured to protect the single-serve portion of honeycomb from impact from at least two directions by creating a frame having at least two sides. In some implementations, the protective support structure may include one or more of a plastic-based material, a paper-based material, and/or other materials. In some implementations, the protective support structure may be water-resistant and/or oil-resistant. In some implementations, a plastic-based material may include one or more of polyethylene, polypropylene, polystyrene, and/or other polyolefins. In some implementations, a plastic-based material may include cellophane and/or other viscose-based materials. The single-serve portion of honeycomb may be contained within and protected from impact by the protective support structure.

One aspect of the disclosure relates to a method for packaging a single-serve portion of honeycomb. The method may comprise obtaining the single-serve portion of honeycomb and containing the single-serve portion of honeycomb in a protective support structure to protect the single-serve portion of honeycomb from impact.

These and other features and characteristics of the present technology, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the invention. As used in the specification and in the claims, the singular form of "a", "an", and "the" include plural referents unless the context clearly dictates otherwise.

### BRIEF DESCRIPTION OF THE DRAWINGS

FIGs. 1A-1B illustrate pieces of honeycomb.
FIGs. 2A-2B-2C illustrate exemplary shapes of a single-serve portion of honeycomb, in accordance with one or more implementations.
FIGs. 3A-3H, 3J, 4A-4H, 4J-4L, 5A-5H, 5J-N, 5P-5R, and 6A-6D illustrate exemplary support structures for packaging honeycomb, wherein only figs 3D-3J, 4D-4L illustrate implementations of the invention as claimed.
FIG. 7 illustrates a method for packaging a single-serve portion of honeycomb, in accordance with one or more implementations.

### DETAILED DESCRIPTION

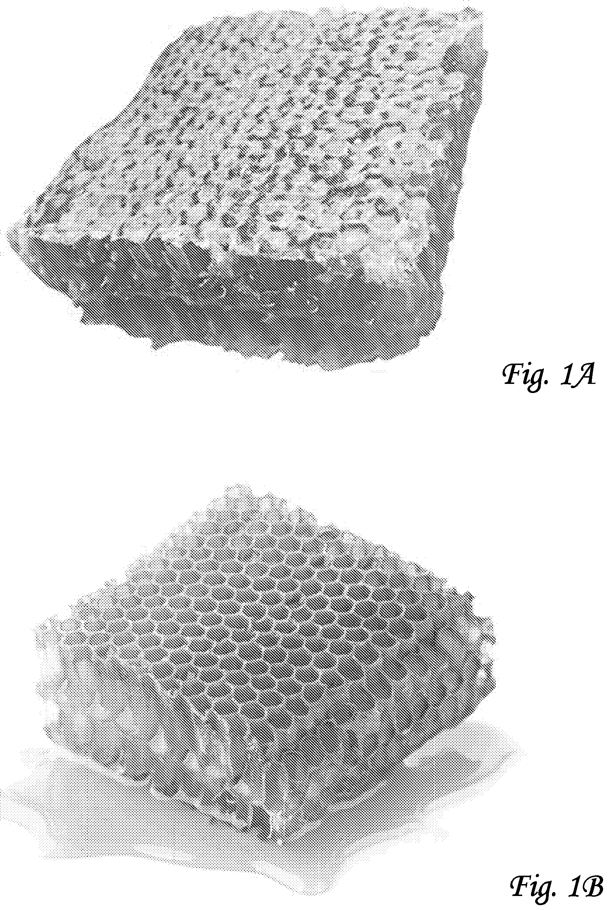

FIG. 1A and 1B illustrate pieces of honeycomb, in this case in the shape of a rectangular prism, a.k.a. cuboid. The height of honeycomb may range from about 0.5 inch to about 2 inches. The structure of honeycomb may be fragile and susceptible to breaking and/or collapsing upon impact, especially impact from a direction perpendicular to the top surface of the honeycomb (i.e., from above in FIG. 1A-1B).

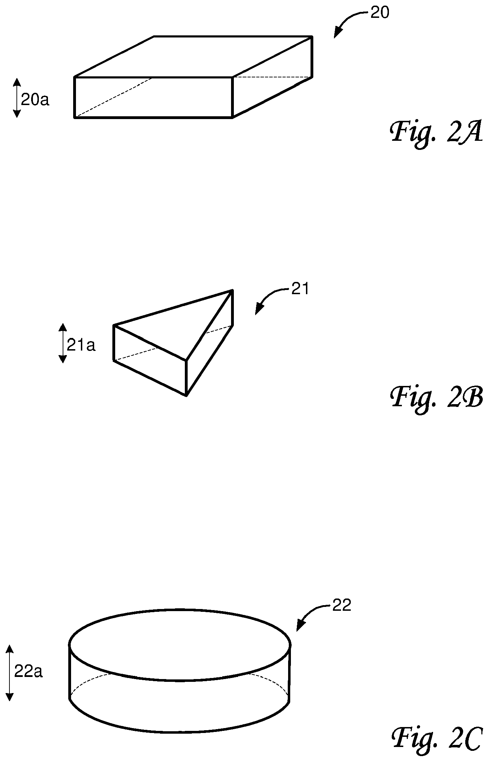

FIGs. 2A-2B-2C illustrate exemplary shapes of a single-serve portion of honeycomb. FIG. 2A illustrates a portion of honeycomb in a shape of a rectangular prism 20, having a height 20a. FIG. 2B illustrates a portion of honeycomb in a shape of a triangular prism 21, having a height 21a. FIG. 2A illustrates a portion of honeycomb in a shape of a cylinder 22, having a height 22a. In some implementations, a single-serve portion of honeycomb may have a different shape than depicted in FIGs 2A-2B-2C. In some implementations, a single-serve portion of honeycomb may have a shape of a polygonal prism having a height between about 0.5 inch and about 2 inches. According to the invention, the single-serve-portion of honeycomb has a volume of about 1 cubic inch (16,4 cubic cm). In some implementations, a single-serve portion of honeycomb may have a number of calories between 50 and 500 calories. For example, the number of calories may be about 50 calories, about 100 calories, about 200 calories, about 300 calories, about 400 calories, about 500 calories, and/or another amount of calories. According to the invention, the single-serve portion of honeycomb weighs about 30 grams.

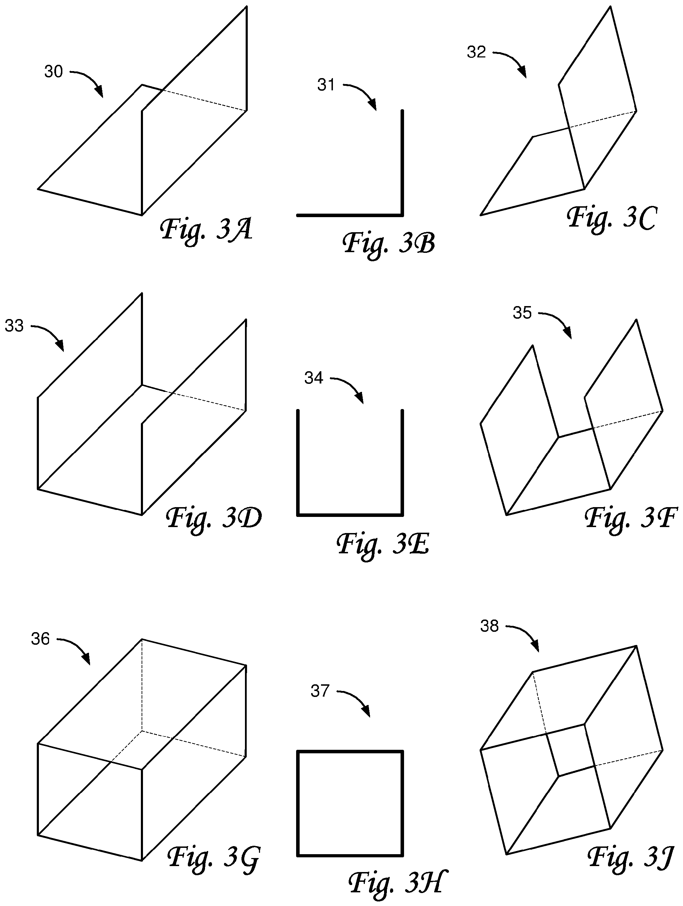

FIGs 3A-3H, 3J illustrate exemplary support structures for packaging honeycomb, in particular for portions of honeycomb in a shape of a rectangular prism. FIG. 3A illustrates a protective support structure 30 (shown isometrically) configured to provide protection from impact from at least two directions, e.g., two perpendicular directions. FIG. 3B illustrates a side-view of a protective support structure 31 configured to provide protection from impact from at least two directions. Protective support structure 31 may be same as or similar to protective support structure 30, though shown from a different view. FIG. 3C illustrates a protective support structure 32 (shown isometrically) configured to provide protection from impact from at least two directions. Protective support structure 32 may be same as or similar to protective support structure 31, though shown from a different view.

In some implementations, a protective support structure may be created by using one or more of the following manufacturing techniques: thermoforming, injection molding, blow molding, rotational molding, heat-shrinking, and/or other manufacturing techniques. By way of non-limiting example, thermoforming is a process in which a plastic sheet is heated to a pliable forming temperature, formed to a specific shape in a mold (or onto a mold), and trimmed to create a usable product. By way of non-limiting example, heat-shrinking may use a thermoplastic resin wrapping film which can be shrunk onto and/or around (food) products.

FIG. 3D illustrates a protective support structure 33 (shown isometrically) configured to provide protection from impact from at least three directions. FIG. 3E illustrates a side-view of a protective support structure 34 configured to provide protection from impact from at least three directions. Protective support structure 34 may be same as or similar to protective support structure 33, though shown from a different view. FIG. 3F illustrates a protective support structure 35 (shown isometrically) configured to provide protection from impact from at least three directions. Protective support structure 35 may be same as or similar to protective support structure 34, though shown from a different view.

FIG. 3G illustrates a protective support structure 36 (shown isometrically) configured to provide protection from impact from at least four directions. Protective support structure 36 may have a shape similar to a sleeve. FIG. 3H illustrates a side-view of a protective support structure 37 configured to provide protection from impact from at least four directions. Protective support structure 37 may be same as or similar to protective support structure 36, though shown from a different view. FIG. 3J illustrates a protective support structure 38 (shown isometrically) configured to provide protection from impact from at least four directions. Protective support structure 38 may be same as or similar to protective support structure 37, though shown from a different view. Additional protective support structures are contemplated within the scope of this disclosure. For example, a protective support structure may be configured to provide protection from impact from at least five directions. For example, a protective support structure may be configured to provide protection from impact from at least six directions. Different protective support structures may be configured to be stackable.

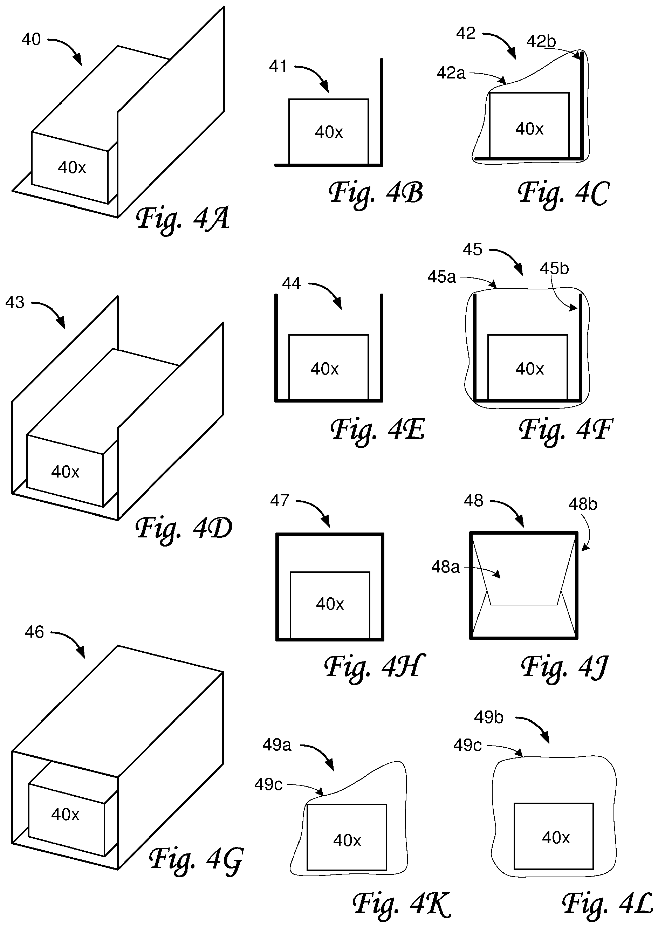

FIGs 4A-4H, 4J-4L illustrate exemplary support structures for packaging a portion of honeycomb 40x. FIG. 4A illustrates a protective support structure 40 (shown isometrically) configured to provide protection to portion of honeycomb 40x from impact from at least two directions, in this case two perpendicular directions. FIG. 4B illustrates a side-view of a protective support structure 41 configured to provide protection. Protective support structure 41 may be same as or similar to protective support structure 40, though shown from a different view. FIG. 4C illustrates a food package 42 that includes portion of honeycomb 40x, a protective support structure 42b, and a wrapper 42a. Protective support structure 42b may be configured to provide protection from impact from at least two directions, in this case two perpendicular directions. Wrapper 42a may be wrapped around portion of honeycomb 40x and protective support structure 42b. Wrapper 42a may include one or more of a plastic-based material, a paper-based material, and/or an aluminum-based material, and/or other materials. In some implementations, wrapper 42a may include food-grade materials. In some implementations, wrapper 42a may be water-resistant and/or oil-resistant. Protective support structure 42b may be same as or similar to protective support structure 41, though shown from a different view.

FIG. 4D illustrates a protective support structure 43 (shown isometrically) configured to provide protection to portion of honeycomb 40x from impact from at least three directions, in this case including two perpendicular directions. FIG. 4E illustrates a side-view of a protective support structure 44 configured to provide protection. Protective support structure 44 may be same as or similar to protective support structure 43, though shown from a different view. FIG. 4D illustrates a food package 45 that includes portion of honeycomb 40x, a protective support structure 45b, and a wrapper 45a. Protective support structure 45b may be configured to provide protection from impact from at least three directions, including two perpendicular directions. Wrapper 45a may be wrapped around portion of honeycomb 40x and protective support structure 45b. Wrapper 45a may include one or more of a plastic-based material, a paper-based material, and/or an aluminum-based material, and/or other materials. In some implementations, wrapper 45a may include food-grade materials. In some implementations, wrapper 45a may be water-resistant and/or oil-resistant. Protective support structure 45b may be same as or similar to protective support structure 44, though shown from a different view. In some implementations, wrapper 45a may undergo heat-shrinking and/or thermoforming. In some implementations, wrapper 45a may be transparent.

FIG. 4G illustrates a protective support structure 46 (shown isometrically) configured to provide protection to portion of honeycomb 40x from impact from at least four directions. FIG. 4H illustrates a side-view of a protective support structure 47 configured to provide protection. Protective support structure 47 may be same as or similar to protective support structure 46, though shown from a different view. FIG. 4J illustrates a food package 48 that includes a protective support structure 48b and a wrapper 45a (a piece of honeycomb may be obscured from view in FIG. 4J). Protective support structure 48b may be configured to provide protection from impact from at least four directions. Wrapper 48a may be wrapped around a portion of honeycomb (not shown) and protective support structure 48b. Wrapper 48a may include one or more of a plastic-based material, a paper-based material, and/or an aluminum-based material, and/or other materials. In some implementations, wrapper 48a may include food-grade materials. In some implementations, wrapper 48a may be water-resistant and/or oil-resistant. Protective support structure 48b may be same as or similar to protective support structure 44, though shown from a different view. In some implementations, wrapper 48a may undergo overwrapping. In some implementations, wrapper 48a may be sealed using an adhesive. In some implementations, nutritional information may be printed on at least one of wrapper 48a and protective support structure 48b. In some implementations, protective support structure 48b may be resealable. In some implementations, protective support structure 48b may form a clamshell that includes a hinge.

FIG. 4K illustrates a side-view of a food package 49a configured to provide protection to portion of honeycomb 40x from impact. Food package 49a includes a protective wrapper 49c. Protective wrapper 49c may be configured to provide protection from impact. Protective wrapper 49c may be wrapped around portion of honeycomb 40x. Protective wrapper 49c may include one or more of a plastic-based material, a paper-based material, and/or an aluminum-based material, and/or other materials. In some implementations, protective wrapper 49c may include food-grade materials. In some implementations, protective wrapper 49c may be water-resistant and/or oil-resistant. In some implementations, protective wrapper 49c may be created by using one or more of the following manufacturing techniques: thermoforming, injection molding, blow molding, rotational molding, heat-shrinking, and/or other manufacturing techniques.

FIG. 4L illustrates a side-view of a food package 49b configured to provide protection to portion of honeycomb 40x from impact. Food package 49b includes a protective wrapper 49c. Protective wrapper 49c may be configured to provide protection from impact. Protective wrapper 49c may be wrapped around portion of honeycomb 40x. Protective wrapper 49c may include one or more of a plastic-based material, a paper-based material, and/or an aluminum-based material, and/or other materials. In some implementations, protective wrapper 49c may include food-grade materials. In some implementations, protective wrapper 49c may be water-resistant and/or oil-resistant. In some implementations, protective wrapper 49c may be created by using one or more of the following manufacturing techniques: thermoforming, injection molding, blow molding, rotational molding, heat-shrinking, and/or other manufacturing techniques.

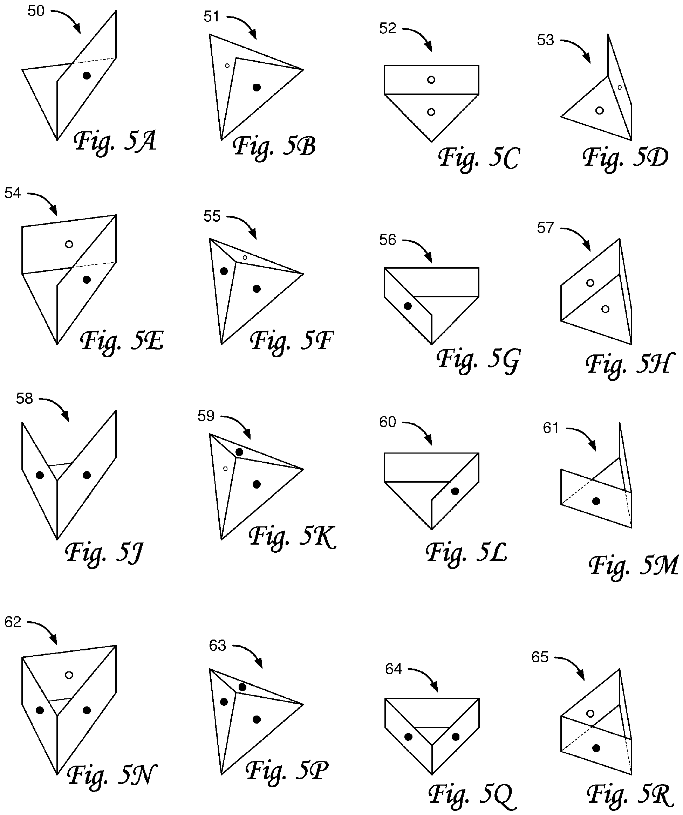

FIGs. 5A-5H, 5J-N, 5P-5R illustrate exemplary support structures for packaging a portion of honeycomb. In some implementations, a wrapper (not depicted in FIGs. 5A-5R, but similar to the depiction in FIG. 4C, 4F, and/or 4J) may be wrapped around a support structure. The small circles depicted within FIGs. 5A-5H, 5J-N, 5P-5R indicate whether a particular surface is facing inwards towards the volume where a portion of honeycomb can be contained (open circle that is not filled in), or outwards (closed circle that is filled in). FIG. 5A illustrates a protective support structure 50 (shown isometrically) configured to provide protection from impact from at least two directions, in this case two perpendicular directions. Protective structure 50 has two sides that create a frame that can protect a portion of honeycomb (*e.g*., a portion in the shape of a triangular prism). FIG. 5E illustrates a protective support structure 54 (shown isometrically) configured to provide protection from impact from at least three directions. Protective support structure 54 may include the same or similar structure as protective support structure 50 plus 1 additional side. FIG. 5J illustrates a protective support structure 58 (shown isometrically) configured to provide protection from impact from at least three directions. Protective support structure 58 may include the same or similar structure as protective support structure 50 plus 1 additional side. FIG. 5N illustrates a protective support structure 62 (shown isometrically) configured to provide protection from impact from at least four directions. Protective support structure 62 may include the same or similar structure as protective support structure 58 plus 1 additional side.

FIG. 5B illustrates a protective support structure 51 (shown isometrically) configured to provide protection from impact from at least two directions, in this case two perpendicular directions. Protective structure 51 has two sides that create a frame that can protect a portion of honeycomb (*e.g*., a portion in the shape of a triangular prism). FIG. 5F illustrates a protective support structure 55 (shown isometrically) configured to provide protection from impact from at least three directions. Protective support structure 55 may include the same or similar structure as protective support structure 51 plus 1 additional side. FIG. 5K illustrates a protective support structure 59 (shown isometrically) configured to provide protection from impact from at least three directions. Protective support structure 59 may include the same or similar structure as protective support structure 51 plus 1 additional side. FIG. 5P illustrates a protective support structure 63 (shown isometrically) configured to provide protection from impact from at least four directions. Protective support structure 63 may include the same or similar structure as protective support structure 55 or 59 plus 1 additional side.

FIG. 5C illustrates a protective support structure 52 (shown isometrically) configured to provide protection from impact from at least two directions, in this case two perpendicular directions. Protective structure 52 has two sides that create a frame that can protect a portion of honeycomb (*e.g*., a portion in the shape of a triangular prism). FIG. 5G illustrates a protective support structure 56 (shown isometrically) configured to provide protection from impact from at least three directions. Protective support structure 56 may include the same or similar structure as protective support structure 52 plus 1 additional side. FIG. 5L illustrates a protective support structure 60 (shown isometrically) configured to provide protection from impact from at least three directions. Protective support structure 60 may include the same or similar structure as protective support structure 52 plus 1 additional side. FIG. 5Q illustrates a protective support structure 64 (shown isometrically) configured to provide protection from impact from at least four directions. Protective support structure 62 may include the same or similar structure as protective support structure 56 or 60 plus 1 additional side.

FIG. 5D illustrates a protective support structure 53 (shown isometrically) configured to provide protection from impact from at least two directions, in this case two perpendicular directions. Protective structure 53 has two sides that create a frame that can protect a portion of honeycomb (*e.g.*, a portion in the shape of a triangular prism). FIG. 5H illustrates a protective support structure 57 (shown isometrically) configured to provide protection from impact from at least three directions. Protective support structure 57 may include the same or similar structure as protective support structure 53 plus 1 additional side. FIG. 5M illustrates a protective support structure 61 (shown isometrically) configured to provide protection from impact from at least three directions. Protective support structure 61 may include the same or similar structure as protective support structure 53 plus 1 additional side. FIG. 5R illustrates a protective support structure 65 (shown isometrically) configured to provide protection from impact from at least four directions. Protective support structure 65 may include the same or similar structure as protective support structure 57 or 61 plus 1 additional side.

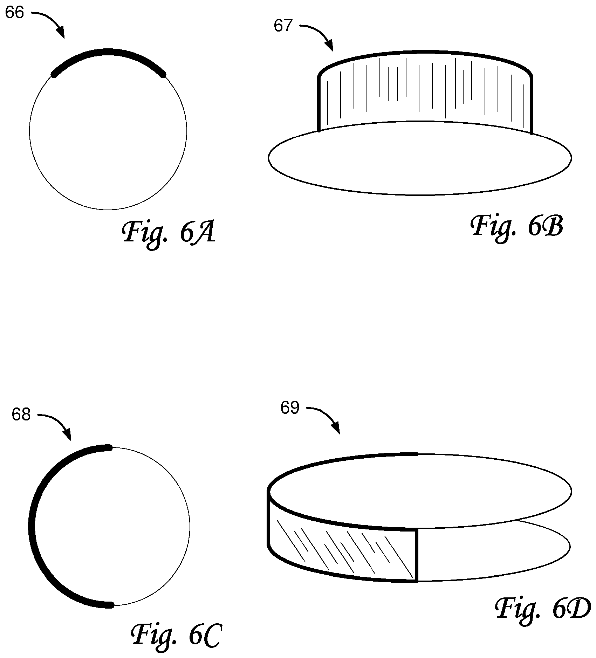

FIGs. 6A-6D illustrate exemplary support structures for packaging a portion of honeycomb. FIG. 6A illustrates a protective support structure 66 (shown from top view) configured to provide protection from impact from at least two directions. Protective structure 60 has two sides that create a frame that can protect a portion of honeycomb (e.g., a portion in the shape of a cylinder). FIG. 6B illustrates a protective support structure 67 (shown isometrically) configured to provide protection from impact from at least two directions. Protective support structure 67 may include the same or similar structure as protective support structure 66, though shown from a different view.

FIG. 6C illustrates a protective support structure 68 (shown from top view) configured to provide protection from impact from at least two directions. Protective structure 68 has two sides that create a frame that can protect a portion of honeycomb (e.g., a portion in the shape of a cylinder). FIG. 6D illustrates a protective support structure 69 (shown isometrically) configured to provide protection from impact from at least two directions. Protective support structure 69 may include the same or similar structure as protective support structure 68, though shown from a different view.

In some implementations, a portion of honeycomb may be protected by a wrapper that includes one or more of a plastic-based material, a paper-based material, and/or other materials. In some implementations, the wrapper may be water-resistant and/or oil-resistant. In some implementations, the wrapper may be created by using one or more of the following manufacturing techniques: thermoforming, injection molding, blow molding, rotational molding, heat-shrinking, and/or other manufacturing techniques. By way of non-limiting example, FIGs. 4K and 4L illustrate such wrappers.

In some implementations, a portion of honeycomb may be chilled and/or frozen prior to being packaged.

In some implementations, a food package such as, by way of non-limiting example, food package 42 may further include one or more supplemental ingredients. The single-serve portion of honeycomb may be combined in a combination with the one or more supplemental ingredients (*e.g*., dietary supplements). In some implementations, a combination may be created through infusion, injection, topical spraying, rolling, and/or other techniques. In some implementations, the one or more supplemental ingredients may include one or more of a flavor ingredient (*e.g*., ginger), a spice ingredient, an ingredient that includes vitamin (*e.g*., vitamin C), a mineral ingredient (*e.g*., zinc), and/or a medicinal ingredient (*e.g*., folic acid). In some implementations, consumption of honeycomb (*e.g*., a combination of honeycomb and one or more supplemental ingredients) may provide health-related and/or functional benefits. In some implementations, a particular supplemental ingredient may have multiple health-related and/or functional benefits. For example, such consumption may provide or aid in cholesterol management and/or liver function. For example, such consumption may be beneficial due to antibacterial and/or antifungal properties. For example, such consumption may have anti-inflammatory benefits. In some implementations, such consumption may treat peptic ulcers. In some implementations, such consumption may provide or aid in weight management, treatment for pollen allergies, and/or treatment for a sore throat. In some implementations, such consumption may reduce the risk of developing diabetes. In some implementations, such consumption may promote restorative sleep. In some implementations, combinations of multiple health-related benefits may be provide to a consumer.

By way of non-limiting example, energy may be increased through one or more of pineapple, ginger, matcha, caffeine, vitamin B12, and/or other supplemental ingredients. By way of non-limiting example, immunity may be enhanced through one or more of orange, lemon, ginger, echinacea, cayenne, reishi mushroom, and/or other supplemental ingredients. By way of non-limiting example, joint pain and/or skin irritations may be improved through one or more of pineapple, chamomile, aloe, turmeric, and/or other supplemental ingredients. By way of non-limiting example, digestive issues may be alleviated through one or more of aloe, ginger, apple cider vinegar, and/or other supplemental ingredients. By way of non-limiting example, liver functionality may be improved through one or more of grapefruit, beet, aloe, burdock root, cayenne, milk thistle, and/or other supplemental ingredients.

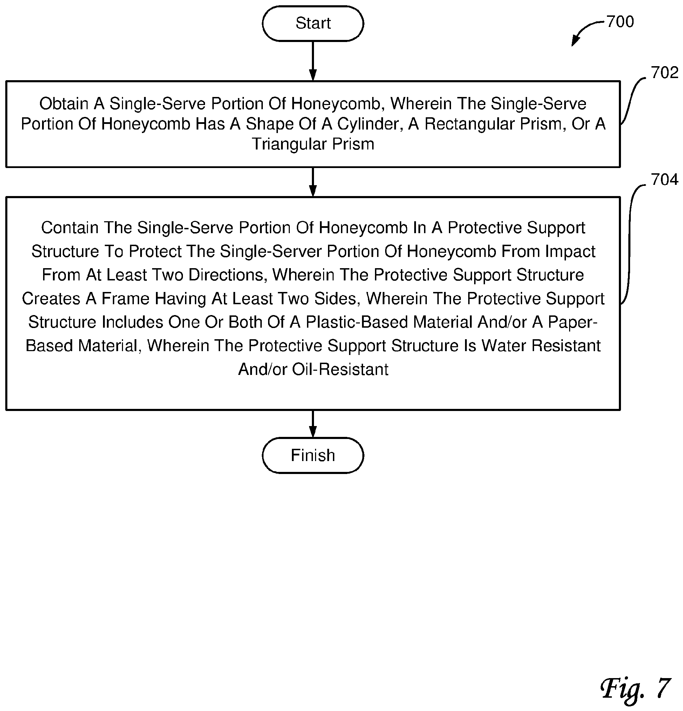

FIG. 7 illustrates a method 700 a method for packaging a single-serve portion of honeycomb. The operations of method 700 presented below are intended to be illustrative. In some embodiments, method 700 may be accomplished with one or more additional operations not described, and/or without one or more of the operations discussed. Additionally, the order in which the operations of method 700 are illustrated in FIG. 7 and described below is not intended to be limiting.

At an operation 702, a single-serve portion of honeycomb is obtained. The single-serve portion of honeycomb may have a shape of a cylinder, a rectangular prism, or a triangular prism. Such operations may be performed by a portion of honeycomb, such as portion of honeycomb 40x in FIGs. 4A-4H, in accordance with one or more implementations.

At an operation 704, the single-serve portion of honeycomb is contained in a protective support structure to protect the single-serve portion of honeycomb from impact from at least two directions. The protective support structure includes one or both of a food-grade plastic-based material and/or a food-grade paper-based material. The protective support structure is water-resistant and/or oil-resistant. Such operations may be performed by a protective support structure, such as protective support structure 40 in FIG. 4A, in accordance with one or more implementations.

## Claims

1. A food package (42, 45, 48, 49a, 49b), comprising:
a single-serve portion of honeycomb having a structure, wherein an amount of honeycomb in the single-serve portion of honeycomb weighs about 30 grams and has a volume of about 1 cubic inch (16,4 cubic cm);
a rigid protective support structure (30, 31, 32, 33, 34, 35, 36, 37, 38, 40, 41, 42b, 43, 44, 45b, 46, 47, 48b, 50, 54, 58, 62, 63, 64, 65, 66, 67, 68, 69) being shaped as a rectangular prism and having at least three orthogonal sides that are orthogonal to each other, wherein the rigid protective support structure has a structural rigidity that protects the structure of the single-serve portion of honeycomb from damage caused by impact from any of at least three orthogonal directions, wherein the rigid protective support structure includes one or both of a food-grade plastic-based material and/or a food-grade paper-based material, wherein the rigid protective support structure is water-resistant and/or oil-resistant,
wherein the structure of the single-serve portion of honeycomb is contained within the rigid protective support structure, and wherein the structure of the single-serve portion of honeycomb is protected by the rigid protective support structure; and
a wrapper (42a, 45a, 48a, 49c) wrapped around at least some of the single-serve portion of honeycomb and the rigid protective support structure, wherein the wrapper (42a, 45a, 48a, 49c) includes one or more of a second food-grade plastic-based material, a second food-grade paper-based material, and/or a food-grade aluminum-based material, wherein the wrapper (42a, 45a, 48a, 49c) is water-resistant and/or oil-resistant.

2. The food package of claim 1, further comprising one or more supplemental ingredients combined in a combination with the single-serve portion of honeycomb, wherein the one or more supplemental ingredients include one or more of a flavor ingredient, a spice ingredient, an ingredient that includes a vitamin, a mineral ingredient, and/or a medicinal ingredient.

3. The food package of claim 3, wherein the one or more supplemental ingredients include the medicinal ingredient, and wherein the combination of the single-serve portion of honeycomb and the medicinal ingredient is configured to provide health-related benefits to a user who consumes the combination.

4. The food package of claim 1, wherein the rigid protective support structure includes polyethylene.

5. The food package of claim 1, wherein the rigid protective support structure is configured to be resealable.

6. The food package of claim 1, wherein the rigid protective support structure forms a clamshell having a hinge.

7. The food package of claim 1, wherein the wrapper is sealed by one or both of heat-shrinking and/or over-wrapping.

8. The food package of claim 1, wherein the wrapper is closed by an adhesive.

9. The food package of claim 1, wherein nutritional information is printed on at least one of the wrapper and the rigid protective support structure.

10. A method for packaging a single-serve portion of honeycomb, the method comprising:
obtaining the single-serve portion of honeycomb having a structure, wherein an amount of honeycomb in the single-serve portion of honeycomb weighs about 30 grams and has a volume of about 1 cubic inch (16,4 cubic cm);
containing the single-serve portion of honeycomb in a rigid protective support structure being shaped as a rectangular prism and having structural rigidity to protect the structure of the single-serve portion of honeycomb from damage caused by impact from any of at least three orthogonal directions, wherein the rigid protective support structure has at least three orthogonal sides that are orthogonal to each other, wherein the rigid protective support structure includes one or both of a food-grade plastic-based material and/or a food-grade paper-based material, wherein the rigid protective support structure is water-resistant and/or oil-resistant; and
wrapping, by a wrapper that is water-resistant and/or oil-resistant, around at least some of the rigid protective support structure, wherein the wrapper includes one or more of a second food-grade plastic-based material, a second food-grade paper-based material, and/or a food-grade aluminum-based material.

## Patentansprüche

1. Lebensmittelverpackung (42, 45, 48, 49a, 49b), umfassend:
einen Einzelportionswabenabschnitt mit einer Struktur, wobei eine Wabenmenge in dem Einzelportionswabenabschnitt etwa 30 Gramm wiegt und ein Volumen von etwa 1 Kubikzoll (16,4 Kubikzentimeter) aufweist;
eine starre, schützende Stütz- bzw. Trägerstruktur (30, 31, 32, 33, 34, 35, 36, 37, 38, 40, 41, 42b, 43, 44, 45b, 46, 47, 48b, 50, 54, 58, 62, 63, 64, 65, 66, 67, 68, 69), die als ein rechteckiges Prisma geformt ist und zumindest drei orthogonale Seiten aufweist, die orthogonal zueinander sind, wobei die starre, schützende Trägerstruktur eine strukturelle Steifigkeit aufweist, die die Struktur des Einzelportionswabenabschnitts vor Schäden durch Aufprall aus zumindest drei orthogonalen Richtungen schützt, wobei die starre, schützende Trägerstruktur eines oder beide eines kunststoffbasierten Materials in Lebensmittelqualität und/oder eines papierbasierten Materials in Lebensmittelqualität enthält, wobei die starre, schützende Trägerstruktur wasserbeständig und/oder ölbeständig ist,
wobei die Struktur des Einzelportionswabenabschnitts in der starren, schützenden Trägerstruktur enthalten ist und wobei die Struktur des Einzelportionswabenabschnitts durch die starre, schützende Trägerstruktur geschützt ist; und
eine Umwicklung (42a, 45a, 48a, 49c), die um zumindest einen Teil des Einzelportionswabenabschnitts und die starre, schützende Trägerstruktur gewickelt ist, wobei die Umwicklung (42a, 45a, 48a, 49c) eins oder mehrere eines zweiten kunststoffbasierten Materials in Lebensmittelqualität, eines zweiten papierbasierten Materials in Lebensmittelqualität und/oder eines aluminiumbasierten Materials in Lebensmittelqualität enthält, wobei die Umwicklung (42a, 45a, 48a, 49c) wasserbeständig und/oder ölbeständig ist.

2. Lebensmittelverpackung nach Anspruch 1, ferner umfassend einen oder mehrere ergänzende Inhaltsstoffe bzw. Bestandteile, die in einer Kombination mit dem Einzelportionswabenabschnitt kombiniert sind, wobei der eine oder mehreren ergänzenden Inhaltsstoffe einen oder mehrere von Geschmacksinhaltsstoff, Gewürzinhaltsstoff, Inhaltsstoff, der ein Vitamin enthält, Mineralstoff und/oder medizinischem Inhaltsstoff enthält.

3. Lebensmittelverpackung nach Anspruch 3, wobei der eine oder die mehreren ergänzenden Inhaltsstoffe den medizinischen Inhaltsstoff umfassen und wobei die Kombination aus dem Einzelportionswabenabschnitt und dem medizinischen Inhaltsstoff konfiguriert ist, einem Benutzer, der die Kombination konsumiert, gesundheitsbezogene Vorteile zu bieten.

4. Lebensmittelverpackung nach Anspruch 1, wobei die starre, schützende Trägerstruktur Polyethylen enthält.

5. Lebensmittelverpackung nach Anspruch 1, wobei die starre, schützende Trägerstruktur konfiguriert ist, wiederverschließbar zu sein.

6. Lebensmittelverpackung nach Anspruch 1, wobei die starre, schützende Trägerstruktur ein Klappgehäuse mit einem Scharnier bildet.

7. Lebensmittelverpackung nach Anspruch 1, wobei die Umwicklung durch eines oder beides von Wärmeschrumpfen und/oder Umwickeln versiegelt wird.

8. Lebensmittelverpackung nach Anspruch 1, wobei die Umwicklung durch einen Klebstoff verschlossen ist.

9. Lebensmittelverpackung nach Anspruch 1, wobei Nährwertinformationen auf zumindest eines von Umwicklung und/oder starre, schützende Trägerstruktur aufgedruckt sind.

10. Verfahren zum Verpacken eines Einzelportionswabenabschnitt, wobei das Verfahren umfasst:
Erhalten des Einzelportionswabenabschnitts mit einer Struktur, wobei eine Wabenmenge in dem Einzelportionswabenabschnitt etwa 30 Gramm wiegt und ein Volumen von etwa 1 Kubikzoll (16,4 Kubikzentimeter) aufweist;
Enthalten bzw. Einschließen des Einzelportionswabenabschnitts in einer starren, schützenden Stütz- bzw. Trägerstruktur, die als ein rechteckiges Prisma geformt ist und strukturelle Steifigkeit aufweist, um die Struktur des Einzelportionswabenabschnitts vor Schäden durch Aufprall aus zumindest drei orthogonalen Richtungen zu schützen, wobei die starre, schützende Trägerstruktur zumindest drei orthogonale Seiten aufweist, die orthogonal zueinander sind, wobei die starre, schützende Trägerstruktur eines oder beide eines kunststoffbasierten Materials in Lebensmittelqualität und/oder eines papierbasierten Materials in Lebensmittelqualität enthält, wobei die starre, schützende Trägerstruktur wasserbeständig und/oder ölbeständig ist, und Umwickeln, mittels einer wasser- und/oder ölbeständigen Umwicklung, um zumindest einen Teil der starren, schützenden Trägerstruktur,
wobei die Umwicklung eins oder mehrere eines zweiten kunststoffbasierten Materials in Lebensmittelqualität, eines zweiten papierbasierten Materials in Lebensmittelqualität und/oder eines aluminiumbasierten Materials in Lebensmittelqualität enthält.

## Revendications

1. Emballage alimentaire (42, 45, 48, 49a, 49b), comprenant :
une portion à une seule utilisation de structure alvéolaire qui a une structure, dans lequel une quantité de structure alvéolaire dans la portion à une seule utilisation pèse environ 30 grammes et a un volume d'environ 1 pouce cube (16,4 cm cubes) ;
une structure de support de protection rigide (30, 31, 32, 33, 34, 35, 36, 37, 38, 40, 41, 42b, 43, 44, 45b, 46, 47, 48b, 50, 54, 58, 62, 63, 64, 65, 66, 67, 68, 69) ayant la forme d'un prisme rectangulaire et ayant au moins trois côtés orthogonaux qui sont orthogonaux les uns par rapport aux autres, dans lequel la structure de support de protection rigide a une rigidité de structure qui protège la structure de la portion à une seule utilisation de structure alvéolaire de dommages causés par un impact provenant de l'une quelconque desdits au moins trois directions orthogonales, dans lequel la structure de support de protection rigide comporte l'un ou les deux parmi un matériau à base de plastique de qualité alimentaire et/ou un matériau à base de papier de qualité alimentaire, dans lequel la structure de support de protection rigide est résistante à l'eau et/ou résistante à l'huile,
dans lequel la structure de la portion à une seule utilisation de structure alvéolaire est contenue à l'intérieur de la structure de support de protection rigide, et dans lequel la structure de la portion à une seule utilisation de structure alvéolaire est protégée par la structure de support de protection rigide ; et
une enveloppe (42a, 45a, 48a, 49c) enveloppée autour d'au moins une partie de la portion à une seule utilisation de structure alvéolaire et de la structure de support de protection rigide, dans lequel l'enveloppe (42a, 45a, 48a, 49c) comporte l'un ou plusieurs parmi un second matériau à base de plastique de qualité alimentaire, un second matériau à base de papier de qualité alimentaire, et/ou un matériau à base d'aluminium de qualité alimentaire, dans lequel l'enveloppe (42a, 45a, 48a, 49c) est résistante à l'eau et/ou résistante à l'huile.

2. Emballage alimentaire selon la revendication 1, comprenant en outre un ou plusieurs ingrédients supplémentaires combinés en une combinaison avec la portion à une seule utilisation de structure alvéolaire, dans lequel les un ou plusieurs ingrédients supplémentaires comportent l'un ou plusieurs parmi un ingrédient de saveur, un ingrédient épicé, un ingrédient qui comporte une vitamine, un ingrédient minéral et/ou un ingrédient médicinal.

3. Emballage alimentaire selon la revendication 3, dans lequel les un ou plusieurs ingrédients supplémentaires comportent l'ingrédient médicinal, et dans lequel la combinaison de la portion à une seule utilisation de structure alvéolaire et de l'ingrédient médicinal est configurée pour fournir des avantages en matière de santé à un utilisateur qui consomme la combinaison.

4. Emballage alimentaire selon la revendication 1, dans lequel la structure de support de protection rigide comporte du polyéthylène.

5. Emballage alimentaire selon la revendication 1, dans lequel la structure de support de protection rigide est configurée pour être refermable.

6. Emballage alimentaire selon la revendication 1, dans lequel la structure de support de protection rigide forme un clapet ayant une charnière.

7. Emballage alimentaire selon la revendication 1, dans lequel l'enveloppe est scellée par l'un ou les deux parmi la thermorétraction et/ou le surenveloppage.

8. Emballage alimentaire selon la revendication 1, dans lequel l'enveloppe est fermée par un adhésif.

9. Emballage alimentaire selon la revendication 1, dans lequel des informations nutritionnelles sont imprimées sur au moins l'une parmi l'enveloppe et la structure de support de protection rigide.

10. Procédé d'emballage d'une portion à une seule utilisation de structure alvéolaire, le procédé comprenant :
l'obtention de la portion à une seule utilisation de structure alvéolaire, dans lequel une quantité de structure alvéolaire dans la portion à une seule utilisation de structure alvéolaire pèse environ 30 grammes et a un volume d'environ 1 pouce cube (16,4 cm cubes) ;
le fait de contenir la portion à une seule utilisation de structure alvéolaire dans une structure de support de protection rigide ayant la forme d'un prisme rectangulaire et ayant une rigidité de structure pour protéger la structure de la portion à une seule utilisation de structure alvéolaire de dommages causés par un impact provenant de l'une quelconque des au moins trois directions orthogonales,
dans lequel la structure de support de protection rigide a au moins trois côtés orthogonaux qui sont orthogonaux les uns par rapport aux autres, dans lequel la structure de support de protection rigide comprend l'un ou les deux parmi un matériau à base de plastique de qualité alimentaire et/ou un matériau à base de papier de qualité alimentaire, dans lequel la structure de support de protection rigide est résistante à l'eau et/ou résistante à l'huile ; et
l'enveloppement, par une enveloppe qui est résistante à l'eau et/ou résistante à l'huile, d'au moins une partie de la structure de support de protection rigide, dans lequel l'enveloppe comporte l'un ou plusieurs parmi un second matériau à base de plastique de qualité alimentaire, un second matériau à base de papier de qualité alimentaire et/ou un matériau à base d'aluminium de qualité alimentaire.
